# EUROPEAN PATENT APPLICATION

(11) **EP 4 803 534 A1**
(43) Date of publication of application: **09.09.2026**
(21) Application number: 24892884.8
(22) Date of filing: 06.08.2024
(51) Int. Cl.: C07K 14/415, C07K 19/00, C12N 15/29

(54) **MUTATED BR2 PROTEIN AND USE THEREOF**

(30) Priority: 22.11.2023 CN 202311559090
(71) Applicant: Shandong Shunfeng Biotechnology Co., Ltd., Jinan, Shandong 250000 (CN)
(72) Inventor: ZHAO, Jun, Jinan, Shandong 250000 (CN); NIU, Xiaomu, Jinan, Shandong 250000 (CN); XIE, Guangning, Jinan, Shandong 250000 (CN)
(74) Representative: Bayramoglu et al.
(86) International application number: PCT/CN2024/109966
(87) International publication number: WO 2025/107720

(57) **Abstract**

A mutated br2 protein is provided, where a CDS sequence and a 3'UTR sequence of the mutated br2 protein include base deletions relative to a CDS sequence and a 3'UTR sequence of a parental br2 protein, where the parental br2 protein is derived from maize. The mutated br2 protein leads to reduced plant height and ear height, improved lodging resistance, and increased yield under high-density planting conditions in maize, and has important application value in maize dwarfing breeding.

## Description

The present application claims priority to Chinese Patent Application CN202311559090.3, filed November 22, 2023. The entire contents of the above-mentioned Chinese patent application are incorporated herein by reference.

### TECHNICAL FIELD

The present invention belongs to the fields of biotechnology and crop genetics and breeding, and relates to a mutated brachytic2 (br2) protein and an application thereof, particularly a mutated br2 protein in maize and an application in reducing maize plant height and ear height and improving maize lodging resistance.

### BACKGROUND TECHNOLOGY

Maize is the most widely planted crop in the world, with an annual global planting area exceeding 2 billion mu and a total output reaching 1 billion tons. Its edible value, industrial value, and feed value make it occupy an important position in China's food security guarantee system. The current increase in maize yield is attributed to increased planting density. Under high-density conditions, plants exhibit a shade-avoidance response, manifesting as changes in traits such as increased plant height and ear height, thinner stems, a smaller stem-leaf angle, elongated leaves, abnormal male and female development, and an extended anthesis-silking interval. Ultimately, this results in reduced lodging resistance, lower photosynthetic efficiency, lower seed setting rate, and lower yield per plant. Varieties with compact plant types and shorter plant height generally exhibit stronger tolerance to high density. Reducing plant height often leads to thicker stems, and lowering the ear position is particularly beneficial for increasing lodging resistance of the plant. Furthermore, appropriately reducing plant height and ear height can also improve the maize harvest index. Therefore, dwarfing breeding while maintaining yield is an important direction in current maize breeding.

Although there is much research on plant height, and plant hormones such as auxins, gibberellins, brassinolide, and cytokinins can regulate plant height, the germplasm resources in maize suitable for dwarfing improvement in plant height are limited. Using limited dwarf germplasm resources for plant height improvement through traditional breeding methods will further narrow the already scarce maize breeding germplasm resources, hindering the development of more superior maize varieties.

Various types of dwarf mutants have been created in maize inbred lines via gene editing technology through methods such as single-gene knockout, alteration of gene amino acid sequences, and regulation of gene expression levels, achieving varying degrees of plant height reduction. Moreover, some mutant forms show a greater reduction in ear height than in plant height; these mutations, which primarily reduce the length of internodes below the ear, are more beneficial for breeding lodging-resistant and high-density-tolerant maize varieties.

### CONTENT OF THE INVENTION

The present invention provides a mutated br2 protein and an application of the mutated protein in reducing maize plant height and ear height, improving maize lodging resistance, increasing maize yield, and increasing maize yield under high-density planting conditions.

In one aspect, the present invention provides a mutated br2 protein, where the coding sequence (CDS) sequence and the 3'untranslated region (UTR) sequence of the mutated br2 protein include base deletions relative to the CDS sequence and the 3'UTR sequence of the parental br2 protein derived from maize; the mutated br2 protein leads to reduced plant height and/or ear height, improved lodging resistance, increased yield, and increased yield under high-density planting conditions in maize.

In one embodiment, the CDS sequence of the mutated br2 protein, relative to the CDS sequence of the parental br2 protein, lacks bases corresponding to positions 4102-4137 of the sequence shown in SEQ ID No. 1; the 3'UTR sequence of the mutated br2 protein, relative to the 3'UTR sequence of the parental br2 protein, lacks bases corresponding to positions 1-36 and positions 160-162 of the sequence shown in SEQ ID No. 2.

In one embodiment, the parental br2 protein is derived from a naturally occurring maize line (e.g., a naturally occurring maize inbred line), and the CDS sequence of the parental br2 protein has at least 80%, at least 85%, at least 90%, at least 95%, at least 96%, at least 97%, at least 98%, and at least 99% sequence identity with the sequence shown in SEQ ID No. 1; the 3'UTR sequence of the parental br2 protein has at least 80%, at least 85%, at least 90%, at least 95%, at least 96%, at least 97%, at least 98%, and at least 99% sequence identity with the sequence shown in SEQ ID No. 2.

In one embodiment, the parental br2 protein is derived from the maize inbred line.

In one embodiment, the parental br2 protein is derived from the maize inbred line PH4CV.

In one embodiment, the maize is the maize inbred line PH4CV.

In one embodiment, the CDS sequence of the parental br2 protein is as shown in SEQ ID No. 1.

In one embodiment, the 3'UTR sequence of the parental br2 protein is shown in SEQ ID No. 2.

In one embodiment, the amino acid sequence of the parental br2 protein is shown in SEQ ID No. 3.

In one embodiment, the CDS sequence of the mutated br2 protein is shown in SEQ ID No. 5.

In one embodiment, the 3'UTR sequence of the mutated br2 protein is shown in SEQ ID No. 6.

In another aspect, a fusion protein is provided, including the above-mentioned mutated protein or a bioactive fragment thereof; further, the fusion protein further includes a protein fused to the mutated protein, such as a tag peptide, a plastid-targeting peptide, or a regulatory element. The tag peptide may be, for example, a histidine tag, 6×His; the plastid-targeting peptide may be, for example, a peptide guided into a chloroplast; the regulatory element may be, for example, a promoter sequence, a terminator sequence, a leader sequence, a polyadenylation sequence, a marker gene, etc.

In another aspect, the present invention provides a polynucleotide encoding the mutated br2 protein or the fusion protein.

In another preferred embodiment, the polynucleotide is selected from the group consisting of genomic sequences, cDNA sequences, RNA sequences, or a combination thereof.

In another preferred embodiment, the polynucleotide is preferably single-stranded or double-stranded.

In another preferred embodiment, the polynucleotide further includes, flanking the open reading frame (ORF) of the mutant polypeptide, an auxiliary element selected from the group consisting of signal peptides, secretory peptides, tag sequences (such as 6His), nuclear localization signals, or a combination thereof.

In another preferred embodiment, the polynucleotide further includes a promoter operatively linked to the ORF sequence of the mutant polypeptide.

In another preferred embodiment, the promoter is selected from the group consisting of constitutive promoters, tissue-specific promoters, inducible promoters, or strong promoters.

In another aspect, the present invention provides a nucleic acid construct, including the polynucleotide and a regulatory element operatively linked thereto.

In another preferred embodiment, the regulatory element is selected from one or more of the group consisting of enhancers, transposons, promoters, terminators, leader sequences, polyadenylic acid sequences, and marker genes.

In another aspect, the present invention also provides a vector, including a nucleic acid sequence encoding the mutated br2 protein of the present invention or the above-mentioned polynucleotide, preferably, the vector further includes an expression regulatory element operatively linked to the above-mentioned nucleic acid sequence.

In another preferred embodiment, the vector includes a cloning vector, an expression vector, a shuttle vector, or an integration vector.

In one embodiment, the vector may be a vector for gene editing of the CDS sequence and/or 3'UTR sequence of an endogenous br2 protein in a host cell.

In one embodiment, the expression vector further includes at least one origin of replication to achieve self-replication.

In one embodiment, the vector may be a vector that is integrated into the genome and replicates along with the chromosome into which it is integrated when introduced into a host cell.

The vector may be of the type plasmid, virus, cosmid, bacteriophage, or the like, which are well known to those skilled in the art.

Preferably, the vector in the present invention is a plasmid.

In another aspect, the present invention provides a host cell, including one or more of the following: the mutated br2 protein, the encoding gene, the fusion protein, the polynucleotide, the nucleic acid construct, and the vector; or, the host cell genome integrates the polynucleotide.

In another preferred embodiment, the host cell is a eukaryotic cell, such as a yeast cell, animal cell, or plant cell.

In another preferred embodiment, the host cell is a prokaryotic cell, such as *Escherichia coli.*

In another preferred embodiment, the plant includes angiosperms and gymnosperms.

In another preferred embodiment, the plant includes monocotyledons and dicotyledons.

In another preferred embodiment, the plant includes herbaceous plants and woody plants.

In another preferred embodiment, the plant includes *Arabidopsis thaliana,* tobacco, rice, maize, sorghum, barley, wheat, millet, soybean, tomato, potato, quinoa, lettuce, rapeseed, Chinese cabbage, and strawberry.

In another aspect, the present invention provides an editing vector system, including one or more vectors, where the one or more vectors at least include a guide sequence targeting the 3'UTR sequence of the parental br2 protein. The guide sequence includes a nucleotide sequence of a portion of the 3'UTR sequence of the parental br2 protein, preferably including at least 15 bp of the nucleotide sequence of the 3'UTR sequence of the br2 protein, more preferably including at least 20 bp of the nucleotide sequence of the 3'UTR sequence of the br2 protein. In one embodiment, the editing vector system further includes a gene-editing enzyme. The gene-editing enzyme includes nucleases from CRISPR (Clustered Regularly Interspaced Short Palindromic Repeats), TALEN (Transcription Activator-like (TAL) effector nucleases), and ZFN (Zinc finger nuclease) editing tools.

Preferably, the gene-editing enzyme is a Cas protein, also known as a CRISPR enzyme or Cas effector protein, including but not limited to: Cas9 protein, Cas12 protein, Cas13 protein, Cas14 protein, Csm1 protein, and FDK1 protein.

Preferably, the Cas protein is operatively linked to a first regulatory element.

In one embodiment, the gene-editing enzyme is a Cas9 protein, and the vector further includes a Scaffold sequence that specifically binds to the Cas9 protein. The Scaffold sequence and the guide sequence are operatively linked to form a guide guide sequence (gRNA). Preferably, the gRNA is operatively linked to a second regulatory element.

In other embodiments, the gene-editing enzyme is a Cas12 protein, for example, Cas12a, Cas12b, Cas12i, or a Cas12i mutant protein, and the vector further includes a direct repeat sequence that specifically binds to the Cas12 protein. The direct repeat sequence and the guide sequence are operatively linked to form a guide guide sequence (gRNA). Preferably, the gRNA is operatively linked to a second regulatory element.

In other embodiments, the gene-editing enzyme is a Cas12i mutant protein, the amino acid sequence of the wild-type Cas12i is shown in SEQ ID No. 4; compared to SEQ ID No. 4, the Cas12i mutant protein has the following mutations: S at position 7 is mutated to R, D at position 233 is mutated to R, D at position 267 is mutated to R, N at position 369 is mutated to R, and S at position 433 is mutated to R.

The regulatory elements include promoters, terminator sequences, leader sequences, polyadenylation sequences, signal peptide coding regions, marker genes, enhancers, internal ribosome entry sites (IRES), and other expression control elements (e.g., transcription termination signals, such as polyadenylation signals and polyU sequences).

Preferably, the editing vector system further includes base editing elements selected from adenine deaminases and/or cytosine deaminases.

In one embodiment, the editing vector further includes a resistance gene for screening purposes. This resistance gene includes hyg, bar, kana, rif, spec, and amp, and is well known to those skilled in the art.

Preferably, the Cas protein is nCas9 or another Cas9 protein with nick activity. Here, "n" represents nick, i.e., a Cas protein with only single-strand cleavage activity.

In another aspect, the present invention provides a gene-editing reagent allowed for producing the above-mentioned mutant polypeptide in a plant. The gene-editing reagent includes a CRISPR/Cas protein and gRNA, where the gRNA is allowed to target the CDS sequence and the 3'UTR sequence of the endogenous br2 protein in the plant. Optionally, the gene-editing reagent further includes a base editing element selected from adenine deaminase and/or cytosine deaminase.

In another embodiment, the gene-editing reagent includes the above-mentioned editing vector system.

In another aspect, the present invention provides a method for reducing plant height and/or ear height of the plant, improving lodging resistance of the plant, increasing yield of the plant, or increasing yield of the plant under high-density planting conditions; or, a method for preparing a plant with reduced plant height and/or ear height, improved lodging resistance, increased yield, or increased yield under high-density planting conditions, the method including the step of introducing the above-mentioned mutated br2 protein into plant cells, plant seeds, plant tissues, plant parts, or the plant, where the plant is maize.

In another aspect, the present invention provides a method for improving plant traits, the method includes the step of introducing the above-mentioned mutated br2 protein into plant cells, plant seeds, plant tissues, plant parts, or the plant, where the plant is maize.

In one embodiment, the improving the plant traits includes reducing plant height and/or ear height of the plant, improving lodging resistance of the plant, increasing yield of the plant, or increasing yield of the plant under high-density planting conditions.

In one embodiment, introducing the mutated br2 protein includes the step of expressing the mutated br2 protein in plant cells, plant tissues, plant parts, or the plant, for example, by expressing the mutated protein using an expression vector, or by integrating the polynucleotide encoding the mutated protein into the plant genome for expression.

In another preferred embodiment, introducing the mutated br2 protein includes the step of mutating the CDS sequence and the 3'UTR sequence of an endogenous br2 protein in the plant to introduce the mutated protein.

In another preferred embodiment, introducing the mutated br2 protein includes the step of mutating and expressing the CDS sequence and the 3'UTR sequence of an endogenous br2 protein in the plant to introduce the mutated protein.

In another preferred embodiment, in the method, the method of introducing the mutation includes natural variation, physical mutagenesis (such as ultraviolet mutagenesis, X-ray or Y-ray mutagenesis), chemical mutagenesis (such as nitrite, hydroxylamine, EMS, nitrosoguanidine, etc.), biological mutagenesis (such as virus- or bacterial-mediated mutagenesis), and gene editing.

In another preferred embodiment, the method includes the following steps:
(1) introducing an expression vector including a gene-editing tool into plant cells, plant tissues, or plant parts;
(2) applying the gene-editing tool to the 3'UTR sequence of its endogenous br2 protein and causing it to mutate at the above-mentioned mutation sites corresponding to SEQ ID No. 1 and SEQ ID No. 2;
(3) screening for mutated plant cells, plant tissues, or plant parts; and
(4) isolating the gene-editing tool.

In another preferred embodiment, the gene-editing tool includes CRISPR, TALEN, and ZFN.

In another aspect, the present invention also provides the application of the above-mentioned mutated br2 protein, polynucleotide, vector, nucleic acid construct, gene-editing reagent, or host cell in preparing the plant with reduced plant height and/or ear height, improved lodging resistance, increased yield, or increased yield under high-density planting conditions, where the plant is maize; or, in preparing a reagent or a kit for reducing plant height and/or ear height of the plant, improving lodging resistance of the plant, increasing yield of the plant, or increasing yield of the plant under high-density planting conditions.

The present invention also provides a maize plant with reduced plant height and/or ear height, improved lodging resistance, increased yield, or increased yield under high-density planting conditions, where the maize plant includes the above-mentioned mutated br2 protein, polynucleotide, vector, gene-editing reagent, or host cell.

The term "reducing plant height and/or ear height of the plant" or "reduced plant height and/or ear height" means that the plant height and/or the ear height of maize plants including the above-mentioned mutated br2 protein, nucleic acid, nucleic acid construct, vector, gene-editing reagent, or host cell are lower than the plant height and/or the ear height of maize plants including the parental br2 protein.

Plant height refers to the height from the ground to the highest point of the maize.

In one embodiment, maize plants including the mutated br2 protein of the present invention have a plant height reduced by approximately 10%-90% compared to the wild-type, for example, 15%, 20%, 25%, 30%, 35%, 40%, 45%, 55%, 60%, 65%, 70%, 75%, 80%, or 85%.

The term "improving lodging resistance of the plant" or "improved lodging resistance" means that maize plants including the above-mentioned mutated br2 protein, nucleic acid, nucleic acid construct, vector, gene-editing reagent, or host cell have stronger lodging resistance than maize plants including the parental br2 protein.

The term "increasing yield of the plant" or "increased yield" means that the yield of maize plants including the above-mentioned mutated br2 protein, nucleic acid, nucleic acid construct, vector, gene-editing reagent, or host cell is higher than the yield of maize plants including the parental br2 protein.

The term "increasing yield of the plant under high-density planting conditions" or "increased yield under high-density planting conditions" means that the yield of maize plants including the above-mentioned mutated br2 protein, nucleic acid, nucleic acid construct, vector, gene-editing reagent, or host cell under high-density planting conditions is higher than the yield of maize plants including the parental br2 protein under high-density planting conditions.

The "high-density planting" refers to a planting density per unit area of land exceeding the optimal planting density, the guiding planting density, the conventional planting density, or the ordinary planting density. For example, in the Huang-Huai region, the ordinary planting density of maize is approximately 4,500 plants per mu, and the ordinary planting density of a few maize varieties reaches 5,000 plants per mu. In one embodiment, the "high-density planting condition" refers to a maize planting density exceeding 4,500 plants per mu; preferably, exceeding 5,000 plants per mu; more preferably, exceeding 5,500 plants per mu.

The present invention also provides a method for reducing plant height and/or ear height of the plant, improving lodging resistance of the plant, increasing yield of the plant, or increasing yield of the plant under high-density planting conditions; or, a method for preparing the plant with reduced plant height and/or ear height, improved lodging resistance, increased yield, or increased yield under high-density planting conditions, the method includes the step of gene editing the CDS sequence and the 3'UTR sequence of the endogenous br2 protein in the plant using the above-mentioned gene-editing reagent, where the plant is maize.

In another aspect, the present invention provides a reagent or kit for reducing plant height and/or ear height of the plant, improving lodging resistance of the plant, increasing yield of the plant, or increasing yield of the plant under high-density planting conditions, where the reagent includes the mutated protein described in the present invention, a polynucleotide encoding the mutated protein, a vector, a nucleic acid construct, a gene-editing reagent, or a host cell.

Unless otherwise defined, the technical and scientific terms used herein have the same meaning as commonly understood by one of ordinary skill in the art.

The term "vector" is an element that allows the vector to be integrated into the host cell genome or to replicate autonomously in the cell independently of the genome. The vector may include any element that ensures self-replication. It usually carries genes that are not part of the central metabolism of the cell and is usually in the form of double-stranded DNA. The choice of vector usually depends on the compatibility of the vector with the host cell into which the vector is to be introduced. If a vector is used, the choice of vector depends on the method for transforming the host cell that is well known to those skilled in the art. For example, a plasmid vector can be used.

The term "3'UTR" refers to the 3' untranslated region or 3' uncoding region of the br2 gene *(Brachytic2)* in maize, and the term "parental 3'UTR" refers to the sequence from which the mutated 3'UTR sequence originates. In a preferred embodiment, the parental 3'UTR is a 3'UTR nucleic acid molecule of the br2 gene that can be found in nature in maize, and its nucleotides can be obtained through genetic engineering techniques such as genome sequencing, polymerase chain reaction (PCR), etc.

As used herein, the term "identity" refers to the sequence matching between two polypeptides or two nucleic acids. When an identical base or amino acid monomer subunit is present at a given position in both sequences being compared (for example, adenine is present at a certain position in each of two DNA molecules, or lysine is present at a certain position in each of two polypeptides), the molecules are identical at that position. The "percentage identity" between two sequences is a function of the number of matching positions shared by the two sequences divided by the number of positions compared × 100. For example, if six out of ten positions in two sequences match, then the two sequences have 60% identity. For example, the DNA sequences CTGACT and CAGGTT have 50% identity (three out of six positions match). Typically, the comparison is performed when aligning the two sequences to achieve maximum identity. Such an alignment can be achieved by employing the method of Needleman et al. (1970) published in J. Mol. Biol., 48: 443-453, which is conveniently implemented via computer programs such as the Align program (DNAstar, Inc.). The percentage identity between two amino acid sequences can also be determined using the algorithm of E. Meyers and W. Miller (Comput. Appl Biosci., 4:11-17 (1988)) integrated into the ALIGN program (version 2.0), employing a PAM120 weighted residue table, a gap length penalty of 12, and a gap penalty of 4. Alternatively, the percentage identity between two amino acid sequences can be determined using the Needleman and Wunsch algorithm (J MoI Biol. 48:444-453 (1970)) in the GAP program integrated into the GCG software package (available at www.gcg.com), employing a Blossum 62 matrix or a PAM250 matrix, with gap weights of 16, 14, 12, 10, 8, 6, or 4, and length weights of 1, 2, 3, 4, 5, or 6.

"Homology" or "identity" can be calculated using known methods including, but not limited to, the following: Computational Molecular Biology (edited by Lesk, A.M.), Oxford University Press, New York (1988); Biocomputing: Informatics and Genome Projects (edited by Smith, D.W.), Academic Press, New York (1993); Computer Analysis of Sequence Data, Part I (edited by Griffin, A.M. and Griffin, H.G.), Humana Press, New Jersey (1994); Sequence Analysis in Molecular Biology (edited by von Heinje, G.), Academic Press (1987); and Sequence Analysis Primer (edited by Gribskov, M. and Devereux, J.), Stockton Press, New York (1991).

The specific amino acid positions (numbers) of the br2 protein described in the present invention are determined by aligning the target amino acid sequences using standard sequence alignment tools, such as the Smith-Waterman algorithm or the CLUSTALW2 algorithm, where the sequence is considered aligned when the alignment score is the highest. The alignment score can be calculated according to the method described in Wilbur, W.J. and Lipman, D.J. (1983), Rapid similarity searches of nucleic acid and protein data banks. Proc. Natl. Acad. Sci. USA, 80:726-730. In the Clustal W2(1.82) algorithm, the default parameters are preferably used: protein gap opening penalty = 10.0; protein gap extension penalty = 0.2; protein matrix = Gonnet; protein/DNA terminal gap = -1; protein/DNA GAPDIST = 4. The AlignX program (part of the vectorNTI group) is preferably used with default parameters suitable for multiple alignments (gap opening penalty: 10og, gap extension penalty 0.05). By aligning the amino acid sequences of br2 proteins from different maize inbred lines or varieties, the specific positions of the amino acids in the br2 proteins of different parents corresponding to SEQ ID No. 3 are determined. Through sequence alignment methods known in the art, those skilled in the art can determine the amino acid correspondence between the br2 protein sequences of different maize varieties and SEQ ID No. 3.

The specific nucleotide (base) positions (numbers) within the 3'UTR described in the present invention are determined by aligning the target nucleotides using standard sequence alignment tools, such as by aligning two sequences with the Smith-Waterman algorithm or the CLUSTALW2 algorithm, where the sequences are considered aligned when the alignment score is the highest. The alignment score can be calculated according to the method described in Wilbur, W.J. and Lipman, D.J. (1983) Rapid similarity searches of nucleic acid and protein data banks. Proc. Natl. Acad. Sci. USA, 80:726-730. In the ClustalW2 (1.82) algorithm, the default parameters are preferably used: protein gap opening penalty = 10.0; protein gap extension penalty = 0.2; protein matrix = Gonnet; protein/DNA end gap = -1; protein/DNA GAPDIST = 4. The AlignX program (part of the vectorNTI group) is preferably used with default parameters suitable for multiple alignments (gap opening penalty: 10og gap extension penalty 0.05). The specific position in the 3'UTR of different parents is determined by comparing the 3' untranslated region or 3' uncoding region (3'UTR) sequence of the br2 gene *(Brachytic2)* of different maize inbred lines or varieties with SEQ ID No. 2 (the 3'UTR sequence of the br2 gene of the maize inbred line PH4CV). Using sequence alignment methods known in the art, those skilled in the art can determine the base correspondence between the 3'UTR sequence of the br2 gene of different maize varieties and SEQ ID No. 2.

The term "plant tissue" or "plant part" includes plant cells, protoplasts, plant tissue culture, plant callus, plant pieces, and plant embryos, pollen, ovules, seeds, leaves, stems, flowers, branches, seedlings, fruits, cores, spikes, roots, root tips, anthers, etc.

The term "plant cell" should be understood as any cells from or found in plants, which can form, for example: undifferentiated tissues such as callus, differentiated tissues such as embryos, plant components, plants, or seeds.

The term "gene editing" technologies include CRISPR technology, TALEN technology, and ZFN technology. CRISPR technology refers to clustered, regularly interspaced short palindromic repeats derived from the immune system of microorganisms. Where gene editing tools include guide RNA, Cas proteins (such as Cas9, Cpf1, Cas12b, etc.). The gene editing tools referred to in TALEN technology are restriction enzymes that can cut specific DNA sequences, which include a TAL effector DNA binding domain and a DNA cleavage domain. The gene editing tools referred to in ZFN technology are also restriction enzymes that can cut specific DNA sequences, which include a zinc finger DNA binding domain and a DNA cleavage domain. It is well known to those skilled in the art that by constructing nucleotides encoding gene editing tools and other regulatory elements into a suitable vector and then transforming cells, the editing of the genome in the cell can be achieved, and the types of editing include gene knockout, insertion, and base editing.

Those skilled in the art will understand that the structure of a protein can be altered without adversely affecting its activity and function. For example, one or more conserved amino acid substitutions can be introduced into the amino acid sequence of a protein without adversely affecting the protein molecule's activity and/or three-dimensional structure. Examples and implementations of conserved amino acid substitutions are familiar to those skilled in the art. Specifically, an amino acid residue can be substituted with another amino acid residue belonging to the same group as the site to be substituted; that is, a nonpolar amino acid residue can replace another nonpolar amino acid residue, a polar uncharged amino acid residue can replace another polar uncharged amino acid residue, a basic amino acid residue can replace another basic amino acid residue, and an acidic amino acid residue can replace another acidic amino acid residue. Such substituted amino acid residues may or may not be encoded by the genetic code. Conservative substitutions where an amino acid is replaced by another amino acid belonging to the same group shall fall within the scope of the present invention, as long as the substitution does not lead to the inactivation of the protein's biological activity. Therefore, the proteins of the present invention may include one or more conserved substitutions in amino acid sequences, preferably generated by substitutions according to Table 1. Additionally, the present invention also covers proteins including one or more other non-conserved substitutions, provided that such non-conserved substitutions do not significantly affect the desired function and biological activity of the proteins of the present invention. Conservative amino acid substitutions may be performed at one or more predicted non-essential amino acid residues. "Non-essential" amino acid residues are amino acid residues that can be altered (deleted, substituted, or replaced) without changing biological activity, while "essential" amino acid residues are required for biological activity. A "conservative amino acid substitution" is a substitution in which an amino acid residue is replaced by an amino acid residue having a similar side chain. Amino acid substitutions may be performed in non-conserved regions of the br2 protein. Generally, such substitutions are not performed on conserved amino acid residues, or on amino acid residues located within conserved motifs, where such residues are required for protein activity. However, those skilled in the art will understand that functional variants may have fewer conserved or non-conserved changes in conserved regions.

It is well known in the art that one or more amino acid residues can be altered (replaced, deleted, truncated, or inserted) from the N-terminus and/or C-terminus of a protein while retaining its functional activity. Therefore, proteins that have one or more amino acid residues altered from the N-terminus and/or C-terminus of the br2 protein while retaining their desired functional activity are also within the scope of the present invention. These alterations can include changes introduced by modern molecular methods such as PCR, which includes PCR amplification that alters or lengthens the protein-coding sequence by means of an oligonucleotide including an amino acid-coding sequence used in the PCR amplification.

It should be recognized that proteins can be altered in various ways, including amino acid substitution, deletion, truncation, and insertion, and methods for such operations are generally known in the art. For example, amino acid sequence variants of the br2 protein can be prepared by mutagenesis of DNA. This can also be accomplished by other forms of mutagenesis and/or by directed evolution, for example, using known mutagenesis, recombination, and/or shuffling methods, combined with relevant screening methods, to perform single or multiple amino acid substitutions, deletions, and/or insertions.

Those skilled in the art will understand that these minor amino acid changes in the br2 protein of the present invention can occur (e.g., naturally occurring mutations) or be generated (e.g., using r-DNA technology) without loss of protein function or activity. If these mutations occur in the catalytic domain, active site, or other functional domains of the protein, the properties of the polypeptide may be altered, but the polypeptide may retain its activity. If the mutations are not located near the catalytic domain, active site, or other functional domains, a smaller impact can be expected.

Those skilled in the art can identify the essential amino acids of the br2 protein using methods known in the art, such as localized mutagenesis, protein evolution, or bioinformatics analysis. The catalytic domain, active site, or other functional domains of the protein can also be determined by physical analysis of the structure, such as by techniques like nuclear magnetic resonance, crystallography, electron diffraction, or photoaffinity labeling, combined with mutations in presumed key site amino acids.

**Table 1**

| Initial residues | Representative substitutions | Preferred substitutions |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The terms "protein," "polypeptide," and "peptide" are used interchangeably in the present invention and refer to polymers of amino acid residues, including polymers in which one or more amino acid residues are chemical analogs of native amino acid residues. The proteins and polypeptides of the present invention can be generated recombinantly or chemically synthesized. The term "mutated protein" or "mutant protein" refers to a protein that, compared to the amino acid sequence of a parent protein, has one or more amino acid residues substituted, inserted, deleted, and/or added. As used herein, the terms "mutated br2 protein," "mutated br2 polypeptide," "mutant br2 polypeptide," "mutant br2 protein," "mutated protein," "mutant polypeptide," etc., are used interchangeably.

The term "encodes" refers to the inherent properties of a specific nucleotide sequence in a polynucleotide, such as a gene, cDNA, or mRNA, as a template for the synthesis of other polymers and macromolecules in biological processes having defined nucleotide sequences (i.e., rRNA, tRNA, and mRNA) or defined amino acid sequences and the biological properties resulting from them. Thus, if the transcription and translation of mRNA corresponding to a gene produces a protein in a cell or other biological system, then the gene encodes that protein.

The term "amino acid" refers to a carboxylic acid including an amino group. All proteins in living organisms are composed of 20 basic amino acids.

The term "plant" is to be understood as any differentiated multicellular organism capable of photosynthesis, including crop plants at any stage of maturity or development, in particular monocots or dicots, vegetable crops, including artichoke, kohlrabi, arugula, leek, asparagus, lettuce (e.g., head lettuce, leaf lettuce, long leaf lettuce), bok choy, malanga, melons (e.g., melon, watermelon, crenshaw, honeydew, roman melon), rape crops (e.g., *Brussels sprouts,* cabbage, cauliflower, broccoli, curly kale, kale, Chinese cabbage, bok choy), cardoon, carrot, napa, okra, onion, celery, parsley, chickpea, European parsnip, chicory, pepper, potato, cucurbit (e.g., zucchini, cucumber, baby zucchini, cushaw, pumpkin), radish, dry bulb onion, rutabaga, purple eggplant (also called eggplant), salsify, endive, scallion, Cichorium endivia, garlic, spinach, green onions, cushaw, greens, beet (sugar beet and mangel), sweet potato, Swiss chard, wasabi, tomato, turnip, and spice; fruits and/or vine crops, such as apple, apricot, cherry, nectarine, peach, pear, plum, prune, cherry, quince, almond, chestnut, hazelnut, pecan, pistachio, walnut, citrus, blueberry, boysenberry, cranberry, ribe nigrum, loganberry, raspberry, strawberry, blackberry, grape, avocado, banana, kiwi, persimmon, pomegranate, pineapple, tropical fruit, pome, melon, mango, papaya, and lychee; field crops such as clover, alfalfa, evening primrose, meadowfoam, corn/maize (fodder corn, sweet corn, popcorn), hops, jojoba, peanut, rice, safflower, small grain cereal crops (barley, oat, rye, wheat, etc.), sorghum, tobacco, kapok, legumes (beans, lentil, pea, soybean), oil plants (oilseed rape, mustard, poppy, olive, sunflower, coconut, castor oil plants, cocoa bean, peanut), Arabidopsis, fiber plants (cotton, flax, hemp, jute), Lauraceae (cinnamon, camphenone), or a plant such as coffee, sugar cane, tea, and natural rubber plants; and/or bedding plants, such as flowering plants, cactus, succulents and/or ornamentals, and trees, such as forests (broadleaf trees and evergreen trees, such as coniferous tree), fruit trees, ornamental trees, and nut-bearing trees, as well as shrubs and other seedlings.

**The main advantages of the present invention are:**
A mutated br2 protein has been screened in the present invention. Compared with wild-type maize plants, maize plants including the mutated br2 protein of the present invention exhibit significantly reduced plant height and ear height, along with improved lodging resistance.

### DESCRIPTION OF THE DRAWINGS

FIG. 1 is a schematic diagram of the gene editing vector used in this embodiment.
FIG. 2 shows a comparison of plant height and ear height between edited plants and wild-type PH4CV maize plants.
FIG. 3 compares plant height (FIG. 3A) and ear height (FIG. 3B) of different mutant lines.
FIG. 4 compares the agronomic traits of edited plants and wild-type PH4CV plants.

### SEQUENCE LISTING

| SEQ ID No. | Description |
|---|---|
| 1 | CDS sequence of the br2 gene in PH4CV |
| 2 | 3'UTR sequence of the br2 gene in PH4CV |
| 3 | Amino acid sequence of the br2 gene in PH4CV |
| 4 | Amino acid sequence of wild-type Cas12i |
| 5 | CDS sequence of the br2 gene in the edited plant |
| 6 | 3'UTR sequence of the br2 gene in the edited plant |

### SPECIFIC IMPLEMENTATIONS

The present invention will be further described below with reference to embodiments. The following description is merely a preferred embodiment of the present invention and is not intended to limit the present invention in any other way. Any person skilled in the art may make equivalent modifications to the above-disclosed technical content to create equivalent embodiments. Any simple modifications or equivalent changes made to the following embodiments based on the technical essence of the present invention without departing from the solution content of the present invention are all within the protection scope of the present invention.

### Example 1: Target Design and Vector Construction

The genomic sequence and amino acid sequence of the *br2* gene were obtained from the NCBI website (https://www.ncbi.nlm.nih.gov). Targets were selected and designed for the coding regions of different domains and 3'UTR sequences to obtain the br2 gene sequence of the maize inbred line PH4CV. The CDS sequence of the br2 gene of the maize inbred line PH4CV is shown in SEQ ID No. 1, and the amino acid sequence is shown in SEQ ID No. 3; the 3'UTR sequence of the br2 gene of the maize inbred line PH4CV is shown in SEQ ID No. 2.
CDS sequence of the br2 gene: 
ATGTCTAGCAGCGACCCGGAGGAGATCAGGGCGCGCGTCGTCGTTCTCGGTTCGCCC
CATGCCGACGGCGGCGACGAGTGGGCCCGGCCCGAGCTCGAGGCCTTCCATCTGCCG
TCTCCCGCCCACCAGCCTCCTGGCTTCCTAGCCGGGCAACCGGAAGCAGCAGAGCA
ACCCACGCTCCCTGCTCCTGCTGGCCGCAGCAGCAGCAACACGCCTACTACATC
TGGCGGTGGCGGCGCTGCTCCTCCTCTTCCTTCTTCGCCTCCCCCTCCGCCGGCTTCT
CTGGAGACCGAGCAGCCGCCCAATGCCAGGCCAGCCTCCGCCGGCGCCAATGACAG
CAAGAAGCCCACCCCGCCCGCCGCCCTGCGCGACCTCTTCCGCTTCGCCGACGGCCT
CGACTGCGCGCTCATGCTCATCGGCACCCTCGGCGCGCTCGTCCACGGCTGCTCGCT
CCCCGTCTTCCTCCGCTTCTTCGCCGACCTCGTCGACTCCTTCGGCTCCCACGCCGAC
GACCCGGACACCATGGTCCGCCTCGTCGTCAAGTACGCCTTCTACTTCCTCGTCGTCG
 
GAGCGGCAATCTGGGCATCCTCGTGGGCAGAGATCTCTTGCTGGATGTGGACCGGCG
AGCGGCAGTCGACGCGGATGCGGATCCGGTACCTGGACGCGGCGCTGCGGCAGGAC
GTGTCCTTCTTCGACACCGACGTGCGGGCCTCGGACGTGATCTACGCCATCAACGCG
GACGCCGTGGTGGTGCAGGACGCCATCAGCGAGAAGCTGGGCAACCTCATCCACTA
CATGGCCACCTTCGTGGCCGGCTTCGTCGTGGGGTTCACGGCCGCGTGGCAGCTGGC
GCTGGTCACGCTGGCCGTGGTGCCGCTCATCGCCGTCATCGGTGGGCTGAGCGCCGC
CGCGCTCGCCAAGCTCTCGTCCCGCAGCCAGGACGCGCTCTCGGGCGCCAGCGGCAT
CGCGGAGCAGGCGCTCGCGCAGATACGGATCGTGCAGGCGTTCGTCGGCGAGGAGC
GCGAGATGCGGGCCTACTCGGCGGCGCTGGCCGTGGCGCAGAGGATCGGCTACCGC
AGCGGCTTCGCCAAGGGGCTCGGCCTCGGCGGCACCTACTTCACCGTCTTCTGCTGC
TACGGGCTCCTGCTCTGGTACGGCGGCCACCTCGTGCGCGCCCAGCACACCAACGGC
GGGCTCGCCATCGCCACCATGTTCTCCGTCATGATCGGCGGACTGGCCCTCGGGCAGT
CGGCGCCGAGCATGGCCGCGTTCGCCAAGGCGCGTGTGGCGGCTGCCAAGATCTTCC
GCATCATCGACCACAGGCCGGGCATCTCCTCGCGCGACGGCGCGGAGCCGGAGTCG
GTGACGGGGCGGGTGGAGATGCGGGGCGTGGACTTCGCGTACCCGTCGCGGCCGGA
CGTCCCCATCCTGCGCGGCTTCTCGCTGAGCGTGCCCGCCGGGAAGACCATCGCGCT
GGTGGGCAGCTCCGGCTCCGGGAAGAGCACGGTGGTGTCGCTCATCGAGAGATTCTA
CGACCCCAGCGCAGGGCAAATCCTGCTGGACGGGCACGACCTCAGGTCGCTGAAGC
TGCGGTGGCTGCGGCGGCAGATCGGGCTGGTGAGCCAGGAGCCGGCGCTGTTCGCG
ACGAGCATCAGGGAGAACCTGCTGCTGGGGCGGGACAGCCAGAGCGCGACGCTGGC
GGAGATGGAGGAGGCGGCCAGGGTGGCCAACGCCCACTCCTTCATCATCAAACTCCC
CGACGGCTACGACACGCAGGTTGGGGAGCGCGGCCTGCAGCTCTCCGGTGGCCAGA
AGCAGCGCATCGCCATCGCCCGCGCCATGCTCAAGAACCCCGCCATCCTGCTGCTGG
ACGAGGCCACCAGCGCGCTGGACTCCGAGTCTGAGAAGCTCGTGCAGGAGGCGCTG
GACCGCTTCATGATCGGGCGCACCACCCTGGTGATCGCGCACAGGCTGTCCACCATC
CGCAAGGCCGACGTGGTGGCCGTGCTGCAGGGCGGCGCCGTCTCCGAGATGGGCGC
GCACGACGAGCTGATGGCCAAGGGCGAGAACGGCACCTACGCCAAGCTCATCCGCA
TGCAGGAGCAGGCGCACGAGGCGGCGCTCGTCAACGCCCGCCGCAGCAGCGCCAGG
CCCTCCAGCGCCCGCAACTCCGTCAGCTCGCCCATCATGACGCGCAACTCCTCCTAC
GGCCGCTCCCCCTACTCCCGCCGCCTCTCCGACTTCTCCACCTCCGACTTCACCCTCT
CCATCCACGACCCGCACCACCACCACCGGACCATGGCGGACAAGCAGCTGGCGTTCC
GCGCCGGCGCCAGCTCCTTCCTGCGCCTCGCCAGGATGAACTCGCCCGAGTGGGCCT
ACGCGCTCGCCGGCTCCATCGGCTCCATGGTCTGCGGCTCCTTCAGCGCCATCTTCGC
CTACATCCTCAGCGCCGTGCTCAGCGTGTACTACGCGCCGGACCCGCGGTACATGAA
GCGCGAGATCGCCAAATACTGCTACCTGCTCATCGGCATGTCCTCCGCGGCGCTGCTG
 
TTCAACACGGTGCAGCACGTGTTCTGGGACACGGTGGGCGAGAACCTGACCAAGCG
GGTGCGCGAGAAGATGTTCGCCGCCGTGCTCCGCAACGAGATCGCCTGGTTCGACGC
GGACGAGAACGCCAGCGCGCGCGTGGCCGCCAGGCTCGCGCTGGACGCCCAGAACG
TGCGCTCCGCCATCGGGGACCGCATCTCCGTCATCGTCCAGAACTCGGCGCTGATGCT
GGTGGCCTGCACCGCGGGGTTCGTCCTCCAGTGGCGCCTCGCGCTCGTGCTCCTCGC
CGTGTTCCCGCTCGTCGTGGGCGCCACCGTGCTGCAGAAGATGTTCATGAAGGGCTT
CTCGGGGGACCTGGAGGCCGCGCACGCCAGGGCGACGCAGATCGCGGGCGAGGCCG
TGGCCAACCTGCGCACCGTGGCCGCGTTCAACGCGGAGCGCAAGATCACGGGGCTG
TTCGAGGCCAACCTGCGCGGCCCGCTCCGGCGCTGCTTCTGGAAGGGGCAGATCGCC
GGCAGCGGCTACGGCGTGGCGCAGTTCCTGCTGTACGCGTCCTACGCGCTGGGGCTG
TGGTACGCGGCGTGGCTGGTGAAGCACGGCGTGTCCGACTTCTCGCGCACCATCCGC
GTGTTCATGGTGCTGATGGTGTCCGCGAACGGCGCCGCCGAGACGCTGACGCTGGCG
CCGGACTTCATCAAGGGCGGGCGCGCGATGCGGTCGGTGTTCGAGACGATCGACCGC
AAGACGGAGGTGGAGCCCGACGACGTGGACGCGGCGCCGGTGCCGGAGCGGCCGA
GGGGCGAGGTGGAGCTGAAGCACGTGGACTTCTCGTACCCGTCGCGGCCGGACATC
CAGGTGTTCCGCGACCTGAGCCTCCGTGCGCGCGCCGGGAAGACGCTGGCGCTGGT
GGGGCCGAGCGGGTGCGGCAAGAGCTCGGTGCTGGCTCTGGTGCAGCGGTTCTACG
AGCCCACGTCCGGGCGCGTGCTCCTGGACGGCAAGGACGTGCGCAAGTACAACCTG
CGGGCGCTGCGGCGCGTGGTGGCGGTGGTGCCGCAGGAGCCGTTCCTGTTCGCGGC
GAGCATCCACGAGAACATCGCGTACGGGCGCGAGGGCGCGACGGAGGCGGAGGTGG
TGGAGGCGGCGGCGCAGGCGAACGCGCACCGGTTCATCGCGGCGCTGCCGGAGGGG
TACCGGACGCAGGTGGGCGAGCGCGGGGTGCAGCTGTCGGGAGGGCAGCGGCAGC
GGATCGCGATCGCGCGCGCGCTAGTGAAGCAGGCGGCCATCGTGCTGCTGGACGAG
GCGACCAGCGCGCTGGACGCCGAGTCGGAGCGGTGCGTGCAGGAGGCGCTGGAGC
GCGCGGGGTCCGGGCGCACCACCATCGTGGTGGCGCACCGGCTGGCCACGGTGCGC
GGCGCGCACACCATCGCGGTCATCGACGACGGCAAGGTGGCGGAGCAGGGGTCGCA
CTCGCACCTGCTCAAGCACCATCCCGACGGGTGCTACGCGCGGATGCTGCAGCTGCA
GCGGCTGACGGGCGCGGCGGCCGGGCCCGGGCCGTCGTCCTCGTGCAACGGGGCCG
CGTAG (SEQ ID No.1);
3'UTR sequence of the br2 gene: 
GACGGAATGGATGGGTTTGGTTCCTCGAGAGATTGATGGGTGAGGAAGCTGAAGCTC
CGGGATCAAATGGTGGTACTCCATGATCGCAACAATGAGGGGGAAAAAAAGAAAGG
AGAAAATACGGTGGTTCATATGATTGTACAATTTGACGATCTGTTTGAGTCGGGGTTTT
AGGATGATGTAAACCTTCACTCGCCTTTTTTTTACTCTTGTTTCTCATCCGCATCAGTAT
CATCTATCTACATACAGTGTCAGAGATGGGAACTGATCCGCATCATCATCTACCTCCCA
 
AGGCACCCCATATTGTATTAATGTACTTAGTTAGCCTGTTTTATATATACTTATAAGTACC
AAATAGCAGAATTTTA (SEQ ID No.2);
Amino acid sequence of the br2 gene:

MSSSDPEEIRARVVVLGSPHADGGDEWARPELEAFHLPSPAHQPPGFLAGQPEAAEQPTL
PAPAGRSSSSNTPTTSGGGGAAPPLPSSPPPPPASLETEQPPNARPASAGANDSKKPTPPAA
LRDLFRFADGLDCALMLIGTLGALVHGCSLPVFLRFFADLVDSFGSHADDPDTMVRLVV
KYAFYFLVVGAAIWASSWAEISCWMWTGERQSTRMRIRYLDAALRQDVSFFDTDVRAS
DVIYAINADAVVVQDAISEKLGNLIHYMATFVAGFVVGFTAAWQLALVTLAVVPLIAVIG
GLSAAALAKLSSRSQDALSGASGIAEQALAQIRIVQAFVVEEREMRAYSAALAVAQRIGY
RSGFAKGLGLGGTYFTVFCCYGLLLWYGGHLVRAQHTNGGLAIATMFSVMIGGLALGQ
SAPSMAAFAKARVAAAKIFRIIDHRPGISSRDGAEPESVTGRVEMRGVDFAYPSRPDVPIL
RGFSLSVPAGKTIALVGSSGSGKSTVVSLIERFYDPSAGQILLDGHDLRSLKLRWLRRQIG
LVSQEPALFATSIRENLLLGRDSQSATLAEMEEAARVANAHSFIIKLPDGYDTQVGERGLQ
LSGGQKQRIAIARAMLKNPAILLLDEATSALDSESEKLVQEALDRFMIGRTTLVIAHRLSTI
RKADVVAVLQGGAVSEMGAHDELMAKGENGTYAKLIRMQEQAHEAALVNARRSSARP
SSARNSVSSPIMTRNSSYGRSPYSRRLSDFSTSDFTLSIHDPHHHRTMADKQLAFRAGA
SSFLRLARMNSPEWAYALAGSIGSMVCGSFSAIFAYILSAVLSVYYAPDPRYMKREIAKYC
YLLIGMSSAALLFNTVQHVFWDTVGENLTKRVREKMFAAVLRNEIAWFDADENASARV
AARLALDAQNVRSAIGDRISVIVQNSALMLVACTAGFVLQWRLALVLLAVFPLVVGATV
LQKMFMKGFSGDLEAAHARATQIAGEAVANLRTVAAFNAERKITGLFEANLRGPLRRCF
WKGQIAGSGYGVAQFLLYASYALGLWYAAWLVKHGVSDFSRTIRVFMVLMVSANGAAE
TLTLAPDFIKGGRAMRSVFETIDRKTEVEPDDVDAAPVPERPRGEVELKHVDFSYPSRPD
IQVFRDLSLRARAGKTLALVGPSGCGKSSVLALVQRFYEPTSGRVLLDGKDVRKYNLRA
LRRVVAVVPQEPFLFAASIHENIAYGREGATEAEVVEAAAQANAHRFIAALPEGYRTQVG
ERGVQLSGGQRQRIAIARALVKQAAIVLLDEATSALDAESERCVQEALERAGSGRTTIVV
AHRLATVRGAHTIAVIDDGKVAEQGSHSHLLKHHPDGCYARMLQLQRLTGAAAGPGPSS
SCNGAA (SEQ ID No.3);

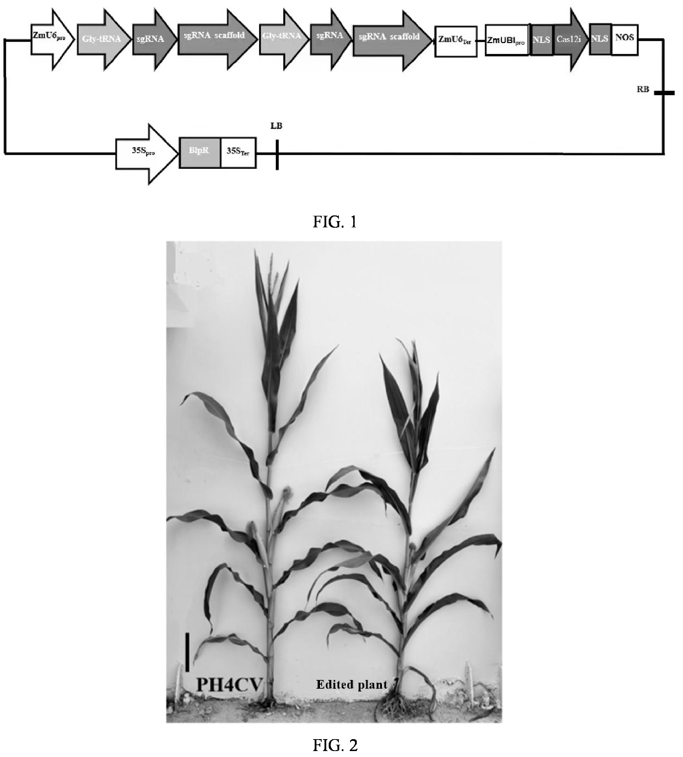

In this embodiment, the 3'UTR of the br2 gene was edited in the maize inbred line PH4CV using the Cas12i mutant protein and sgRNA targeting the 3'UTR sequence of *br2.* The specific operation method can be performed according to conventional methods in the art. A schematic diagram of the constructed gene editing vector in this embodiment is shown in FIG. 1. Where, ZmU6ₚᵣₒ is the U6 promoter, gly-tRNA is glycine tRNA, ZmU6_{Ter} is the terminator, UBIₚᵣₒ is the UBI promoter, NLS is the nuclear localization signal, and Cas12i is the Cas12i mutant protein. In this embodiment, wild-type Cas12i is Cas12f.4 from CN111757889B, and its amino acid sequence is shown in SEQ ID No. 4. Compared with SEQ ID No. 4, the Cas12i mutant protein has the following mutations: S at position 7 is mutated to R, D at position 233 is mutated to R, D at position 267 is mutated to R, N at position 369 is mutated to R, and S at position 433 is mutated to R.
Amino acid sequence of wild-type Cas12i:

MKKVEVSRPYQSLLLPNHRKFKYLDETWNAYKSVKSLLHRFLVCAYGAVPFNKFVEVV
EKVDNDQLVLAFAVRLFRLVPVESTSFAKVDKANLAKSLANHLPVGTAIPANVQSYFDS
NFDPKKYMWIDCAWEADRLAREMGLSASQFSEYATTMLWEDWLPLNKDDVNGWGSV
SGLFGEGKKEDRQQKVKMLNNLLNGIKKNPPKDYTQYLKILLNAFDAKSHKEAVKNYK
GDSTGRTASYLSEKSGEITELMLEQLMSNIQRDIGDKQKEISLPKKDVVKKYLESESGVP
YDQNLWSQAYRNAASSIKKTDTRNFNSTLEKFKNEVELRGLLSEGDDVEILRSKFFSSEF
HKTPDKFVIKPEHIGFNNKYNVVAELYKLKAEATDFESAFATVKDEFEEKGIKHPIKNILE
YIWNNEVPVEKWGRVARFNQSEEKLLRIKANPTVECNQGMTFGNSAMVGEVLRSNYVS
KKGALVSGEHGGRLIGQNNMIWLEMRLLNKGKWETHHVPTHNMKFFEEVHAYNPSLA
DSVNVRNRLYRSEDYTQLPSSITDGLKGNPKAKLLKRQHCALNNMTANVLNPKLSFTIN
KKNDDYTVIIVHSVEVSKPRREVLVGDYLVGMDQNQTASNTYAVMQVVKPKSTDAIPFR
NMWVRFVESGSIESRTLNSRGEYVDQLNHDGVDLFEIGDTEWVDSARKFFNKLGVKHK
DGTLVDLSTAPRKAYAFNNFYFKTMLNHLRSNEVDLTLLRNEILRVANGRFSPMRLGSLS
WTTLKALGSFKSLVLSYFDRLGAKEMVDKEAKDKSLFDLLVAINNKRSNKREERTSRIAS
SLMTVAQKYKVDNAVVHVVVEGNLSSTDRSASKAHNRNTMDWCSRAVVKKLEDMCN
LYGFNIKGVPAFYTSHQDPLVHRADYDDPKPALRCRYSSYSRADFSKWGQNALAAVVR
WASNKKSNTCYKVGAVEFLKQHGLFADKKLTVEQFLSKVKDEEILIPRRGGRVFLTTHRL
LAESTFVYLNGVKYHSCNADEVAAVNICLNDWVIPCKKKMKEESSASG (SEQ ID No.4);

Specifically, in this embodiment, sgRNAs were designed using target Design (http://skl.scau.edu.cn/targetdesign/). The gRNAs targeting the 3'UTR sequence are as follows:
g3'UTR-1 (AGCTTCCTCACCCATCAATC);
g3'UTR-2 (GACGATCTGTTTGAGTCGGG).
g3'UTR-1 and g3'UTR-2 were combined to construct the vector P1515, with the two gRNAs separated by a glycine tRNA.

The specific construction method is as follows:
1) The target fragment g3'UTR-1&2 was amplified using primer pairs, and the recovered fragment was digested with BsaI enzyme;
2) The backbone vector P0522 was digested with BsaI enzyme to recover the 23 kb fragment;
3) The g3'UTR-1&2 was ligated with P0522 to construct the final vector P1515;
4) The above ligation products were transformed into *E. coli* competent cells Trans-T1, respectively, the resulting cells were then plated on Kan plates and cultured. 8 colonies were picked and cultured in liquid medium for 2 h. Bacterial solution PCR was performed, and 2 positive single clones were selected and submitted for sequencing.
5) The single clones with correct sequencing results were selected for propagation, strain preservation, and plasmid extraction, and then transformation into Agrobacterium EHA105. 5 single colonies were picked for culture, and the positive strains were preserved for later use after PCR verification.

### Example 2: Genetic Transformation

### 2.1 Transformation of Agrobacterium

The vector from Example 1 was transformed into Agrobacterium strain EHA105 using a heat shock method. Single clones were picked, cultured in liquid medium, and identified by PCR, then stored at -80°C freezer for later use.

### 2.2 Strain Activation

The strain was removed from the freezer and streaked onto yeast extract peptone (YEP) solid medium.

### 2.3 Preparation of Agrobacterium Infection Solution

Fresh bacterial cells were scraped from the newly activated bacterial plate and resuspended in the infection solution.

### 2.4 Collection of Maize Immature Embryos

Maize ears approximately 10 days after pollination were collected. The husks and silks were removed, and the immature embryos were picked and placed in infection medium (without Agrobacterium) including acetosyringone (AS).

### 2.5 Infection

The immature embryos to be transformed were washed three times with the infection solution until the infection solution was clear. The infection solution was then drained, and 1 mL of bacterial solution was added. The mixture was gently inverted 10 times and allowed to stand for 5-10 minutes. The clean petri dish was taken, 3 sheets of sterile filter paper were placed on the clean petri dish. After completion of infection, the mixture was inverted a few times, and the bacterial solution was quickly poured onto the filter paper. The petri dish was held and rotated to allow uniform distribution of the bacterial solution carrying the immature embryos on the filter paper.

### 2.6 Co-culture

When the bacterial solution was no longer visible on the top layer of filter paper, tweezers were used to lift the top layer of filter paper, placing the side with the immature embryos onto the co-culture medium. Air bubbles between the filter paper and the medium were removed with tweezers, and then a corner of the filter paper was quickly peeled off with tweezers. The immature embryos remaining on the filter paper were transferred to the medium using an embryo removal knife, with the immature embryo shield facing up, followed by incubating in the dark at 22°C for 3 days.

### 2.7 Recovery Culture

After 3 days of co-culture, the immature embryos were transferred to recovery medium.

### 2.8 Differentiation

Immature embryos transformed with the P1515 vector were directly differentiated on differentiation medium.

### 2.9 Rooting

The differentiated seedlings were transferred to rooting medium, with 3-4 seedlings per bottle, and cultured at 25-28°C under light until they grow into complete plants. White roots will grow after 7 days of rooting culture, and samples can be taken for testing.

### Example 3: Screening of Positive Seedlings and Phenotyping of Edited Plants

For regenerated seedlings, a small number of leaves were used to extract genomic DNA using the TPS method.

For plants transformed with the vector, primers were used for detection. If the target product could be amplified, the regenerated seedling was considered a transgenic positive seedling.

For transgenic positive seedlings, primers were used to amplify the corresponding fragment of the br2 gene. The amplified product was sequenced using the Sanger method to confirm the editing pattern. If the sequencing result showed a double peak, the PCR product was ligated into a T vector, and five clones were selected for sequencing to confirm the editing pattern.

The 3'UTR sequence of the br2 gene in the inbred line PH4CV was edited using P1515 to transform the immature embryos of PH4CV. Edited plants were obtained, with the following editing results: deletion of bases 4102-4137 in the CDS region of the br2 gene, deletion of bases 1-36 and 160-162 in the 3'UTR sequence; based on amino acid structure prediction, amino acids 1368-1378 of the br2 protein were deleted, and the protein was extended by 45 amino acids.
CDS sequence of the br2 gene in the edited plant: 
ATGTCTAGCAGCGACCCGGAGGAGATCAGGGCGCGCGTCGTCGTTCTCGGTTCGCCC
CATGCCGACGGCGGCGACGAGTGGGCCCGGCCCGAGCTCGAGGCCTTCCATCTGCCG
TCTCCCGCCCACCAGCCTCCTGGCTTCCTAGCCGGGCAACCGGAAGCAGCAGAGCA
ACCCACGCTCCCTGCTCCTGCTGGCCGCAGCAGCAGCAGCAACACGCCTACTACATC
TGGCGGTGGCGGCGCTGCTCCTCCTCTTCCTTCTTCGCCTCCCCCTCCGCCGGCTTCT
CTGGAGACCGAGCAGCCGCCCAATGCCAGGCCAGCCTCCGCCGGCGCCAATGACAG
CAAGAAGCCCACCCCGCCCGCCGCCCTGCGCGACCTCTTCCGCTTCGCCGACGGCCT
CGACTGCGCGCTCATGCTCATCGGCACCCTCGGCGCGCTCGTCCACGGCTGCTCGCT
CCCCGTCTTCCTCCGCTTCTTCGCCGACCTCGTCGACTCCTTCGGCTCCCACGCCGAC
GACCCGGACACCATGGTCCGCCTCGTCGTCAAGTACGCCTTCTACTTCCTCGTCGTCG
 
GAGCGGCAATCTGGGCATCCTCGTGGGCAGAGATCTCTTGCTGGATGTGGACCGGCG
AGCGGCAGTCGACGCGGATGCGGATCCGGTACCTGGACGCGGCGCTGCGGCAGGAC
GTGTCCTTCTTCGACACCGACGTGCGGGCCTCGGACGTGATCTACGCCATCAACGCG
GACGCCGTGGTGGTGCAGGACGCCATCAGCGAGAAGCTGGGCAACCTCATCCACTA
CATGGCCACCTTCGTGGCCGGCTTCGTCGTGGGGTTCACGGCCGCGTGGCAGCTGGC
GCTGGTCACGCTGGCCGTGGTGCCGCTCATCGCCGTCATCGGTGGGCTGAGCGCCGC
CGCGCTCGCCAAGCTCTCGTCCCGCAGCCAGGACGCGCTCTCGGGCGCCAGCGGCAT
CGCGGAGCAGGCGCTCGCGCAGATACGGATCGTGCAGGCGTTCGTCGGCGAGGAGC
GCGAGATGCGGGCCTACTCGGCGGCGCTGGCCGTGGCGCAGAGGATCGGCTACCGC
AGCGGCTTCGCCAAGGGGCTCGGCCTCGGCGGCACCTACTTCACCGTCTTCTGCTGC
TACGGGCTCCTGCTCTGGTACGGCGGCCACCTCGTGCGCGCCCAGCACACCAACGGC
GGGCTCGCCATCGCCACCATGTTCTCCGTCATGATCGGCGGACTGGCCCTCGGGCAGT
CGGCGCCGAGCATGGCCGCGTTCGCCAAGGCGCGTGTGGCGGCTGCCAAGATCTTCC
GCATCATCGACCACAGGCCGGGCATCTCCTCGCGCGACGGCGCGGAGCCGGAGTCG
GTGACGGGGCGGGTGGAGATGCGGGGCGTGGACTTCGCGTACCCGTCGCGGCCGGA
CGTCCCCATCCTGCGCGGCTTCTCGCTGAGCGTGCCCGCCGGGAAGACCATCGCGCT
GGTGGGCAGCTCCGGCTCCGGGAAGAGCACGGTGGTGTCGCTCATCGAGAGATTCTA
CGACCCCAGCGCAGGGCAAATCCTGCTGGACGGGCACGACCTCAGGTCGCTGAAGC
TGCGGTGGCTGCGGCGGCAGATCGGGCTGGTGAGCCAGGAGCCGGCGCTGTTCGCG
ACGAGCATCAGGGAGAACCTGCTGCTGGGGCGGGACAGCCAGAGCGCGACGCTGGC
GGAGATGGAGGAGGCGGCCAGGGTGGCCAACGCCCACTCCTTCATCATCAAACTCCC
CGACGGCTACGACACGCAGGTTGGGGAGCGCGGCCTGCAGCTCTCCGGTGGCCAGA
AGCAGCGCATCGCCATCGCCCGCGCCATGCTCAAGAACCCCGCCATCCTGCTGCTGG
ACGAGGCCACCAGCGCGCTGGACTCCGAGTCTGAGAAGCTCGTGCAGGAGGCGCTG
GACCGCTTCATGATCGGGCGCACCACCCTGGTGATCGCGCACAGGCTGTCCACCATC
CGCAAGGCCGACGTGGTGGCCGTGCTGCAGGGCGGCGCCGTCTCCGAGATGGGCGC
GCACGACGAGCTGATGGCCAAGGGCGAGAACGGCACCTACGCCAAGCTCATCCGCA
TGCAGGAGCAGGCGCACGAGGCGGCGCTCGTCAACGCCCGCCGCAGCAGCGCCAGG
CCCTCCAGCGCCCGCAACTCCGTCAGCTCGCCCATCATGACGCGCAACTCCTCCTAC
GGCCGCTCCCCCTACTCCCGCCGCCTCTCCGACTTCTCCACCTCCGACTTCACCCTCT
CCATCCACGACCCGCACCACCACCACCGGACCATGGCGGACAAGCAGCTGGCGTTCC
GCGCCGGCGCCAGCTCCTTCCTGCGCCTCGCCAGGATGAACTCGCCCGAGTGGGCCT
ACGCGCTCGCCGGCTCCATCGGCTCCATGGTCTGCGGCTCCTTCAGCGCCATCTTCGC
CTACATCCTCAGCGCCGTGCTCAGCGTGTACTACGCGCCGGACCCGCGGTACATGAA
GCGCGAGATCGCCAAATACTGCTACCTGCTCATCGGCATGTCCTCCGCGGCGCTGCTG
 
TTCAACACGGTGCAGCACGTGTTCTGGGACACGGTGGGCGAGAACCTGACCAAGCG
GGTGCGCGAGAAGATGTTCGCCGCCGTGCTCCGCAACGAGATCGCCTGGTTCGACGC
GGACGAGAACGCCAGCGCGCGCGTGGCCGCCAGGCTCGCGCTGGACGCCCAGAACG
TGCGCTCCGCCATCGGGGACCGCATCTCCGTCATCGTCCAGAACTCGGCGCTGATGCT
GGTGGCCTGCACCGCGGGGTTCGTCCTCCAGTGGCGCCTCGCGCTCGTGCTCCTCGC
CGTGTTCCCGCTCGTCGTGGGCGCCACCGTGCTGCAGAAGATGTTCATGAAGGGCTT
CTCGGGGGACCTGGAGGCCGCGCACGCCAGGGCGACGCAGATCGCGGGCGAGGCCG
TGGCCAACCTGCGCACCGTGGCCGCGTTCAACGCGGAGCGCAAGATCACGGGGCTG
TTCGAGGCCAACCTGCGCGGCCCGCTCCGGCGCTGCTTCTGGAAGGGGCAGATCGCC
GGCAGCGGCTACGGCGTGGCGCAGTTCCTGCTGTACGCGTCCTACGCGCTGGGGCTG
TGGTACGCGGCGTGGCTGGTGAAGCACGGCGTGTCCGACTTCTCGCGCACCATCCGC
GTGTTCATGGTGCTGATGGTGTCCGCGAACGGCGCCGCCGAGACGCTGACGCTGGCG
CCGGACTTCATCAAGGGCGGGCGCGCGATGCGGTCGGTGTTCGAGACGATCGACCGC
AAGACGGAGGTGGAGCCCGACGACGTGGACGCGGCGCCGGTGCCGGAGCGGCCGA
GGGGCGAGGTGGAGCTGAAGCACGTGGACTTCTCGTACCCGTCGCGGCCGGACATC
CAGGTGTTCCGCGACCTGAGCCTCCGTGCGCGCGCCGGGAAGACGCTGGCGCTGGT
GGGGCCGAGCGGGTGCGGCAAGAGCTCGGTGCTGGCTCTGGTGCAGCGGTTCTACG
AGCCCACGTCCGGGCGCGTGCTCCTGGACGGCAAGGACGTGCGCAAGTACAACCTG
CGGGCGCTGCGGCGCGTGGTGGCGGTGGTGCCGCAGGAGCCGTTCCTGTTCGCGGC
GAGCATCCACGAGAACATCGCGTACGGGCGCGAGGGCGCGACGGAGGCGGAGGTGG
TGGAGGCGGCGGCGCAGGCGAACGCGCACCGGTTCATCGCGGCGCTGCCGGAGGGG
TACCGGACGCAGGTGGGCGAGCGCGGGGTGCAGCTGTCGGGAGGGCAGCGGCAGC
GGATCGCGATCGCGCGCGCGCTAGTGAAGCAGGCGGCCATCGTGCTGCTGGACGAG
GCGACCAGCGCGCTGGACGCCGAGTCGGAGCGGTGCGTGCAGGAGGCGCTGGAGC
GCGCGGGGTCCGGGCGCACCACCATCGTGGTGGCGCACCGGCTGGCCACGGTGCGC
GGCGCGCACACCATCGCGGTCATCGACGACGGCAAGGTGGCGGAGCAGGGGTCGCA
CTCGCACCTGCTCAAGCACCATCCCGACGGGTGCTACGCGCGGATGCTGCAGCTGCA
GCGGCTGACGGGCGCGGCGGCCGGG (SEQ ID No.5);
3'UTR sequence of the br2 gene in the edited plant:

TGGGTGAGGAAGCTGAAGCTCCGGGATCAAATGGTGGTACTCCATGATCGCAACAAT
GAGGGGGAAAAAAAGAAAGGAGAAAATACGGTGGTTCATATGATTGTACAATTTGAC
GATCTGTTTTCGGGGTTTTAGGATGATGTAAACCTTCACTCGCCTTTTTTTTACTCTTG
TTTCTCATCCGCATCAGTATCATCTATCTACATACAGTGTCAGAGATGGGAACTGATCC
GCATCATCATCTACCTCCCAAGGCACCCCATATTGTATTAATGTACTTAGTTAGCCTGTT
TTATATATACTTATAAGTACCAAATAGCAGAATTTTA (SEQ ID No.6);

Compared to wild-type PH4CV plants, the edited plants showed a reduction of approximately 20% in plant height and a reduction of approximately 30% in ear height, as shown in FIG. 2.

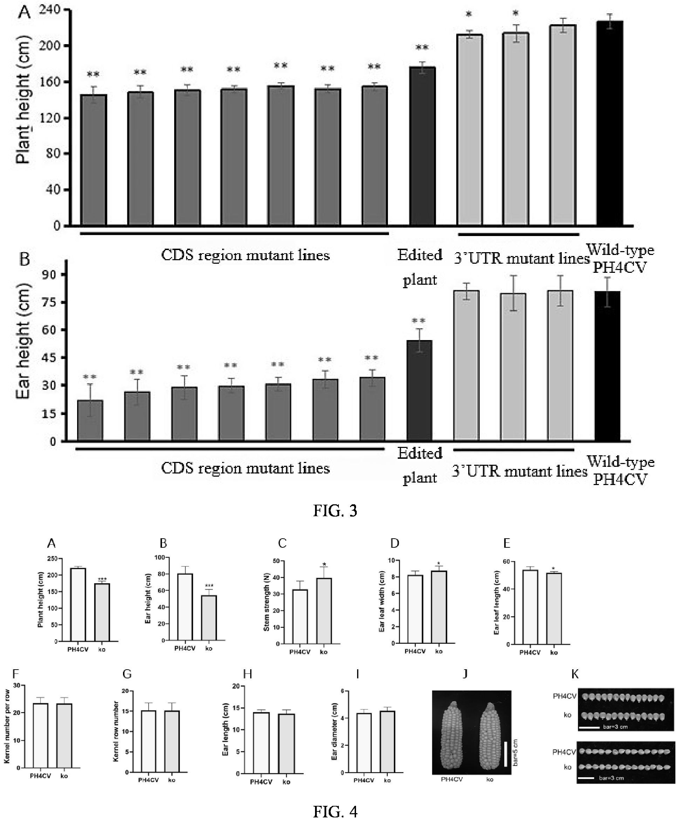

Statistical analysis of the dwarfing phenotypes of lines with different br2 gene mutation types revealed that the plant height of most br2 gene CDS region mutant lines was reduced by more than 30%, and ear height by more than 55%; while the plant height of most br2 gene 3'UTR sequence mutant lines was reduced by less than 10%, with no significant reduction in ear height. Therefore, compared to the dwarfing phenotype (plant height and ear height) of the br2 gene CDS region mutant lines, the dwarfing phenotype of this edited plant is more moderate; compared to the dwarfing phenotype (plant height and ear height) of the br2 gene 3'UTR sequence mutant lines, the dwarfing phenotype of this edited plant is more pronounced, as shown in FIG. 3 (FIG. 3A compares plant height of lines with different mutation types, FIG. 3A compares ear height of each mutation type).

The stem strength of wild-type PH4CV plants and edited plants was determined using a YYD-1B stem strength tester from Zhejiang Top Cloud-Agri Technology Co., Ltd. Fifteen plants from each pollinated line were selected, and the stems were fixed together with the instrument using rubber bands and placed 20 cm above the ground. The corn stems were pushed at a right angle until the stems formed a 45° angle with the ground, and the value was recorded in Newtons (N). This value is the bending resistance, which represents the stem strength. The greater the stem strength, the stronger the plant's resistance to lodging and breakage.

The agronomic traits of the edited plants are shown in FIG. 4. In FIGS. 4A-K, PH4CV refers to wild-type PH4CV plants, and ko refers to edited plants. Compared to the wild-type PH4CV, the plant height was reduced by 20% (FIG. 4A), and the ear height was reduced by 31% (FIG. 4B); stem strength was significantly increased (FIG. 4C), enhancing resistance to lodging and breakage; leaf width was increased (FIG. 4D), while leaf length was shortened (FIG. 4E); there were no significant differences in the number of kernels per row (FIG. 4F) and the number of rows per ear (FIG. 4G); ear length (FIG. 4H and FIG. 4J) was slightly shorter but not significantly different; ear diameter (FIG. 4I) was slightly increased but not significantly different; and kernel width and kernel length (FIG. 4K) were also not significantly different. This indicates that the edited plant not only reduced plant height and ear height but also enhanced lodging resistance, demonstrating potential for lodging tolerance under high-density planting and for increased yield and resistance to lodging under high-density planting conditions.

All references mentioned in the present invention are incorporated herein by reference as if each reference were individually incorporated by reference. Furthermore, it should be understood that after reading the above teachings of the present invention, those skilled in the art can make various alterations or modifications to the present invention, and these equivalent forms also fall within the scope defined by the appended claims of the present invention.

## Claims

1. A mutated br2 protein, wherein a coding sequence (CDS) sequence and a 3'untranslated region (3'UTR) sequence of the mutated br2 protein comprise base deletions relative to a CDS sequence and a 3'UTR sequence of a parental br2 protein; and
the parental br2 protein is derived from maize; and the mutated br2 protein leads to reduced plant height and/or ear height, improved lodging resistance, increased yield, or increased yield under high-density planting conditions in maize.

2. The mutated protein according to claim 1, wherein the CDS sequence of the mutated br2 protein, relative to the CDS sequence of the parental br2 protein, lacks bases corresponding to positions 4102-4137 of the sequence shown in SEQ ID No. 1; the 3'UTR sequence of the mutated br2 protein, relative to the 3'UTR sequence of the parental br2 protein, lacks bases corresponding to positions 1-36 and positions 160-162 of the sequence shown in SEQ ID No. 2;
preferably, the CDS sequence of the parental br2 protein is as shown in SEQ ID No. 1, and the 3'UTR sequence of the parental br2 protein is as shown in SEQ ID No. 2.

3. A polynucleotide encoding the mutated br2 protein according to any one of claims 1-2.

4. A vector, comprising the polynucleotide according to claim 3.

5. A host cell, comprising the mutated br2 protein according to any one of claims 1-2, or a polynucleotide according to claim 3, or a vector according to claim 4.

6. A gene-editing reagent, wherein the gene-editing reagent is allowed for producing the mutated protein according to any one of claims 1-2 in a plant; and the gene-editing reagent comprises a clustered regularly interspaced short palindromic repeats (CRISPR)/CRISPR-associated (Cas) protein and guide RNA (gRNA), wherein the gRNA is allowed to target a 3'UTR sequence of an endogenous br2 protein in the plant, and the plant is maize.

7. A method for improving plant traits, comprising a step of introducing the mutated br2 protein according to any one of claims 1-2 into plant cells, plant seeds, plant tissues, plant parts, or a plant, wherein the plant is maize; and
preferably, the improving the plant traits comprises reducing plant height and/or ear height of the plant, improving lodging resistance of the plant, increasing yield of the plant, or increasing yield of the plant under the high-density planting conditions.

8. The method according to claim 7, wherein introducing the mutated br2 protein according to any one of claims 1-2 comprises a step of mutating a CDS sequence and a 3'UTR sequence of an endogenous br2 protein in the plant to introduce the mutated protein; and preferably, the mutated br2 protein is introduced into the plant via gene editing.

9. The method according to claim 7, wherein introducing the mutated br2 protein according to any one of claims 1-2 comprises a step of expressing the mutated protein in the plant cells, the plant seeds, the plant tissues, the plant parts, or the plant.

10. An application of the mutated protein according to any one of claims 1-2, a polynucleotide according to claim 3, a vector according to claim 4, a host cell according to claim 5, or a gene-editing reagent according to claim 6 in preparing a plant with the reduced plant height and/or ear height, the improved lodging resistance, the increased yield, or the increased yield under the high-density planting conditions; or, in preparing a reagent or a kit for reducing plant height and/or ear height of the plant, improving lodging resistance of the plant, increasing yield of the plant, or increasing yield of the plant under the high-density planting conditions; wherein the plant is maize.
